# EUROPEAN PATENT APPLICATION

(11) **EP 4 721 625 A1**
(43) Date of publication of application: **08.04.2026**
(21) Application number: 25205639.5
(22) Date of filing: 30.09.2025
(51) Int. Cl.: A47C 7/54

(54) **ARMREST DEVICE**

(30) Priority: 04.10.2024 SE 2430507
(71) Applicant: Rini Ergoteknik AB, 186 42 Vallentuna (SE)
(72) Inventor: Egnell, Sven, 186 42 Vallentuna (SE); Svensson, Lars, 186 42 Vallentuna (SE); Weiss, Peter, 186 42 Vallentuna (SE)
(74) Representative: Heimdal, Pär

(57) **Abstract**

The present disclosure provides an armrest device (2) for an operator chair (1), comprising an armrest plate (2a), an upper arm (6a, 6b), a lower arm (5a, 5b), and a clamp (12) for attachment to a support structure. The device includes multiple joints (3, 3a, 4, 13, 14, 15, 16) connecting components, at least one gas cylinder (7, 8) for enabling or disabling movements, and a lock/ release system (21) to control the gas cylinder's mode. A first control mechanism (9) on the armrest plate (2a) allows user operation without hand removal, controlling the lock/ release system (21) to set the gas cylinders (7, 8) in released or locked mode.

## Description

### Technical Field

The present invention generally relates to an adjustable armrest device for operator chairs where there is a requirement for an operator to be able to set the positions of the armrest device with a high degree of freedom in three dimensions without the operator's arm, wrist or hand must leave the armrest plate.

### Background

There is a need for adjustable armrest devices for chairs in many applications within healthcare, laboratories and in the manufacturing industry. In addition, office chairs and special chairs for disabled persons may also be equipped with adjustable armrest devices. The term operator chair therefore refers here to all possible applications of an operator chair.

Previously known adjustable armrests devices come in many varieties where the adjustability is achieved by various methods such as ball joints or movable adjustable joints that are locked with rotating knobs and other type of fasteners. Common for all of them is that the range of adjustments are limited and/ or it is necessary for the operator to remove the hand from the armrest, to adjust one or several controllers on the chair, each associated with a joint. In such case the operator must interrupt the work, which may be in a sterile state, to perform the adjustment of the armrest device position. Alternatively, adjustment of the armrest requires assistance of another person.

Electrical adjustment of the armrest device is another option. These are expensive and are requiring electric power and have their limitations in range of adjustment and are not suited to be adapted directly to chairs.

Mechanical armrest devices are available in a variety of designs and complexity. The ones with lowest complexity have one or two actuable/ adjustable joints that can be adjusted by pressing and releasing a button or loosening and tightening a screw. The adjustment is then up to two movement joints allowing adjustment up/ down and forward/ backwards. The adjustment is done by pressing the button or loosening the screw with one hand and adjusting the position of the armrest device with the other hand and then locking it in the desired position. This simple design is known e.g. from office chairs where the armrest adjustment is made once or seldom and is more intended to fit the operator's constitution or base work position. These solutions are inappropriate in sterile environments, and/ or when frequent adjustments of the armrest are required.

It would be advantageous to achieve a convenient solution overcoming, or at least alleviating, at least some of the above-mentioned drawback(s). It would be desirable to provide a stable armrest device that does not require electrical operation or computer support, with a large range of movements in X-, Y- and Z direction. To better address this concern, a device having the features defined in the independent claims are provided.

### Object of the invention

The invention comprises an armrest device that does not require electrical operation or computer support, with a large range of movement in X-, Y- and Z direction.

The armrest device can be released, moved in X-, Y- and Z direction and locked together in any position without the operator's arm, wrist or hand having to leave the armrest plate.

A further purpose of the armrest device is that the angle of the armrest plate up and down can be released and locked in any position without the operator's arm, wrist or hand having to leave the armrest plate.

A further purpose is to provide an armrest device that is compact in physical size and can easily be adapted to different types of operator's chairs and applications.

A further purpose is to provide an armrest device that easily can be tailored to the body constitution and work preferences of an operator due to a rotating joint at the armrest plate that can be set with a variable level of friction.

A further purpose is to provide an armrest device that is cost-effective to manufacture and requires a minimal amount of maintenance and calibration.

### Summary

The armrest device addresses several features when a steady hand is required and there is a requirement from an operator to be able to change the position of the arms, wrist or hand during work procedure without leaving the armrest plate thus having an automated adjustment feature.

In a first aspect, an armrest device for an operator chair is provided. The armrest device comprises an armrest plate, upper and lower arms, and a clamp for attaching/ securing the armrest device to an operator chair's support structure. The armrest device also comprises multiple joints connecting the armrest plate, arms, and bracket. At least one gas cylinder is designed to allow movement of the armrest plate and arms when in released mode, and to prevent movement when in locked mode. A lock/ release system is employed to switch the gas cylinder between released and locked modes. Additionally, a first control mechanism, situated on the armrest plate for easy access, operates through the lock/ release system to adjust the gas cylinder's mode without requiring the user to lift his/ her hand from the armrest plate. The armrest device provides a highly adjustable and ergonomic support for operators, allowing for precise positioning and stability during procedures. The location of the control mechanism on the armrest plate itself enables easy and efficient adjustment without the need to remove the hand from the armrest, enhancing user comfort and operational efficiency.

The armrest device may comprise a first gas cylinder and a second gas cylinder.

The inclusion of two gas cylinders allows for more complex and precise adjustments of the armrest device, providing greater flexibility in positioning.

The lock/ release system and the first control mechanism are configured to simultaneously set the first gas cylinder and the second gas cylinder in either released mode, or locked mode.

This simultaneous control of multiple gas cylinders simplifies the adjustment process for the user, allowing for quick and efficient repositioning of the armrest device.

The first gas cylinder may be configured to allow adjustment of the inclination of the lower arm when set in the released mode, and the second gas cylinder may be configured to allow adjustment of the inclination of the upper arm when set in the released mode.

This configuration enables independent adjustment of the lower and upper arms, providing a wide range of possible positions to accommodate various user needs and procedural requirements.

The lower arm of the armrest device may comprise a lower arm top and lower arm bottom arranged in a lower parallelogram and the upper arm may comprise an upper arm top and an upper arm bottom, arranged in an upper parallelogram.

Optionally, the armrest device may further comprise a rotation joint at the clamp of the armrest device, wherein the rotation joint is allowed to rotate when set in a released mode, and to disallow rotation when set in a locked mode, and wherein the lock/ release system and the first control mechanism are configured to set the rotation joint in either released mode, or locked mode.

The addition of a controllable rotation joint further enhances the versatility of the armrest device, allowing for rotational adjustments in addition to linear and angular adjustments.

The lock/ release system and the first control mechanism may be configured to simultaneously set the first gas cylinder, the second gas cylinder and the rotation joint in either released mode, or locked mode.

This simultaneous control of all adjustable components through a single mechanism greatly simplifies the operation of the armrest device, allowing for quick and comprehensive adjustments with minimal effort.

Optionally, the rotational resistance of the rotation joint may be adjustable.

Adjustable rotational resistance allows for customization of the rotation joint's behaviour, accommodating different user preferences and task requirements.

The armrest device may further comprise a third gas cylinder, configured to enable tilt angle adjustment of the armrest plate, and a second control mechanism located at the armrest plate for adjusting tilt angle of the armrest plate by setting the third gas cylinder in either released mode, wherein the tilt angle of the armrest plate is adjustable, or in locked mode, wherein the tilt angle of the armrest plate is locked.

The addition of a separately controllable tilt adjustment for the armrest plate provides an extra dimension of customization, allowing users to fine-tune the angle of support for their arm and hand.

The clamp of the armrest device may be configured to be removably attached to a rail of the support structure of the chair.

This removable attachment feature enhances the versatility of the armrest device, allowing it to be easily transferred between different chairs or support structures, or removed when not needed.

The lock/ release system may comprise a hydraulic system in some embodiments, or a wire system in other embodiments.

Thanks to the features of the armrest device, a stable and safe support for an operator's arm is provided. The control mechanism/s enable/s the operator to adjust the armrest device in a convenient and hygienic manner. A surgeon etc., is thereby enabled to adjust the armrest device on an ongoing basis without compromising the sterile coverage.

The adjustment range is sufficiently large in the X-, Y- and Z directions to cover different applications, not only in surgery, but more widely in healthcare, laboratories and in the manufacturing industry and thus be able to handle many possible work positions.

The mechanical nature of the provided solution involving the lock/ release system, and the gas cylinders support an armrest device that is beneficial in terms of size, weight and costs, in comparison with electrically/ computationally controlled armrest devices.

### Brief description of drawings

Figure 1 shows an operator chair, equipped with a left and right armrest device embodied according to the invention, with an operator in the chair.

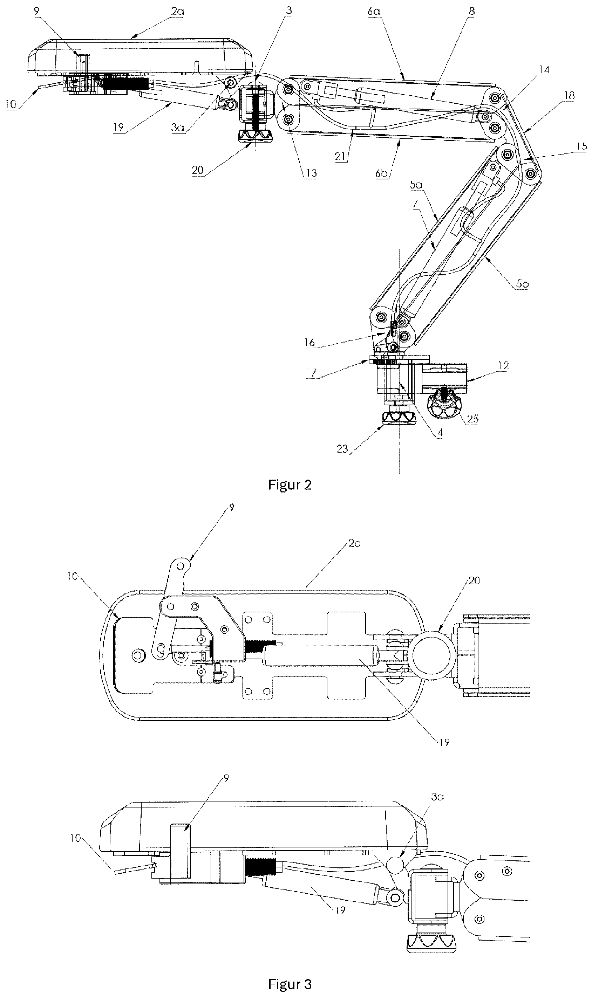

Figure 2 shows an overview of an embodiment of a complete armrest device according to the invention to be attached to an operator chair.
Figure 3 shows an embodiment of the armrest plate seen from underneath and from the side with one control mechanism to be used with the thumb and one control mechanism to be used by the fingers.

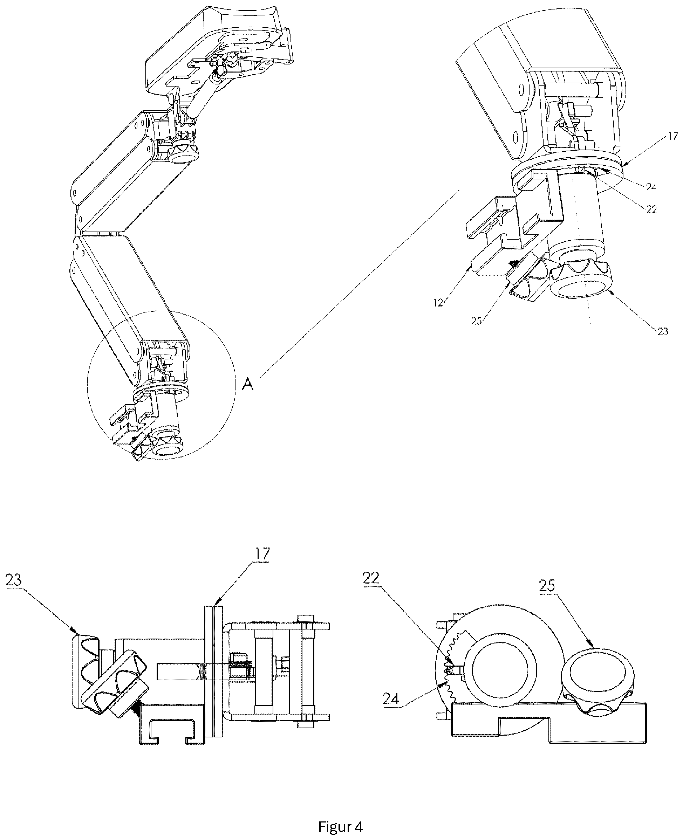

Figure 4 shows an overview and detail of an embodiment where the armrest device is attached to the operator chair.

### Detailed description

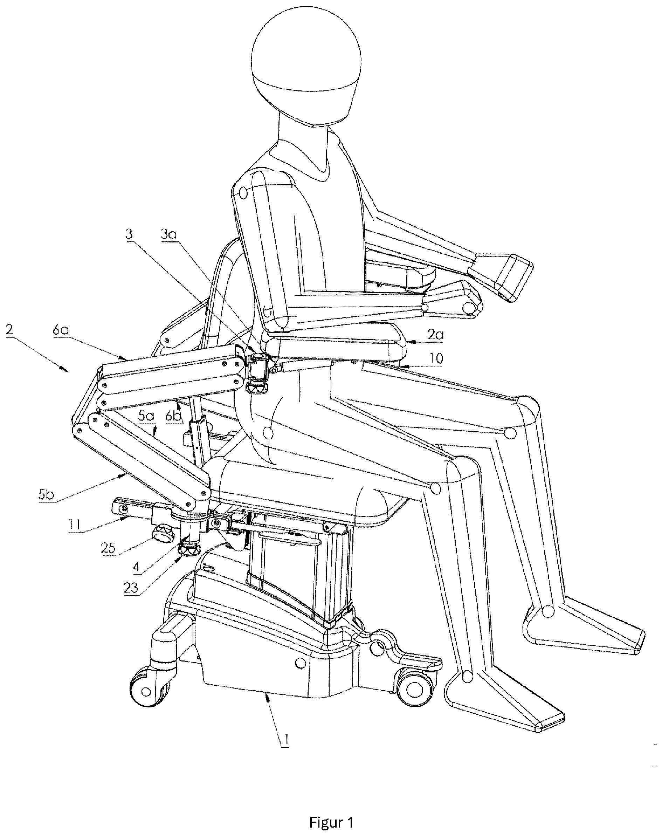

Figure 1 illustrates an armrest device 2 for an operator chair 1 with an automatic adjustment feature in X-, Y- and Z direction using a single control mechanism. This could for example be an armrest device for a surgeon in microsurgery, for a dentist or any operator that needs support of arm, wrist or hand during work. Specifically for a surgeon in microsurgery armrest devices are critical. The armrest device 2 allows the user/ surgeon himself to adjust the working position of the arm, wrist or hand in X- Y- and Z directions during the work without having to take the help of another person or interrupting the work for manual adjustments of the armrest device 2.

In one embodiment, the release and lock of the selected position of the armrest device 2 may be done with only one control mechanism, i.e. the first control mechanism 9 that is placed in connection with the armrest plate 2a where the user easily may find and activate the first control mechanism 9 with the thumb or other finger. The first control mechanism 9 is configured to open/ close apertures of a first gas cylinder 7 and a second gas cylinder 8 via a lock/ release system 21, and thereby either enable or disable movements of the first gas cylinder 7 and the second gas cylinder 8 and thereby also the armrest device 2.

When the first control mechanism 9 is activated and the armrest device 2 is set in a released state, the operator can move the position of the armrest plate 2a to a new desired position with a light hand force. When the desired position is reached, the first control mechanism 9 may be deactivated, and the armrest device 2 may be locked in its new position.

In another embodiment, the angle of the armrest plate 2a relative to the rest of the armrest device 2 may be adjusted by a second control mechanism 10, distinct from the first control mechanism 9. The second control mechanism 10 may be designed as a release lever which is activated with a finger of the user while keeping his/ her hand on the armrest plate 2a. In the activated position it may release the locking of a third gas cylinder 19 which controls the angle of the armrest plate 2a.

In yet another embodiment, the armrest device 2 may have a physical size that is similar to more basic prior art armrest devices and can therefore be used in most relevant spaces and has a flexible fastener mechanism so that it can be applied to different types of operator chairs 1.

In another embodiment the armrest device 2 can be tailored to the body constitution and work preferences of an operator by a first rotating joint 3. This can move the armrest plate 2a inwards and outwards towards the operator and the resistance can be regulated with a rotating knob 20 from loose to fully fixed.

The regulation in X-, Y- and Z direction of the armrest device 2 as well as the angulation of the armrest plate 2a may be done by hydraulic control via the lock/ release system 21 of the tuned gas cylinders 7, 8. The involved components are inexpensive, light weight and require a minimal amount of maintenance and calibration in comparison with for example applying electrical control via electrical motors acting on the armrest device 2. Thereby, the lock/ release system 21 may comprise a hydraulic system connecting the first control mechanism 9 with the first gas cylinder 7 and the second gas cylinder 8, thereby either simultaneously setting the first gas cylinder 7 and the second gas cylinder 8 in released mode by opening the gas cylinders 7, 8 for air flow and enabling an armrest plate 2a to be freely moved by adjusting positions of the upper arm 6a, 6b and the lower arm 5a, 5b, when the first control mechanism 9 is depressed. Correspondingly, when the first control mechanism 9 is released, the first gas cylinder 7 and the second gas cylinder 8 are simultaneously set into locked mode, disabling air to enter or leave the air cylinders 7, 8 thereby locking the position of the armrest plate 2a. The lock/ release system 21 may alternatively comprise a wire system, operating correspondingly as the described hydraulic system, but with a wire connecting the first control mechanism 9 with respective air opening of the first gas cylinder 7 and the second gas cylinder 8.

The armrest device 2 comprises the movable armrest plate 2a which can be adjusted in wide X-, Y- and Z directions, in relation to the other parts of the armrest device 2. The armrest device 2, in the illustrated embodiment is attached to the operator chair 1. The release and locking of the armrest device 2 are done with the first control mechanism 9 located on the armrest plate 2a. The armrest plate 2a can also be angled up and down and locked at the desired angle at the first rotation joint 3 with the second control mechanism 10. Both control mechanisms 9 and 10 can be accessed without the operator's arm, wrist or hand having to leave the armrest plate 2a.

The degree of freedom of movement of the operator chair 1 and the armrest devices 2 on the left and right side are described from the operator's perspective. This means that the operator sits in the chair 1 as illustrated in figure 1. Direction indications are thus entered in the ways that the operator would have entered them. The X- direction is forward and backward in the direction the operator's chest and back is facing, the Y-direction is up and down vertical from the floor and the Z-direction is rotating towards or out from the operator respectively defined in the same way from the operator's perspective.

The operator chair 1 may be designed in such a way that several different parts of the chair 1 can be adjusted to a desired working position, this to provide support for different parts of the operator's body in a desired way. The adjustability of the seat cushion, backrest and height are examples of such adjustment options that exist but are not described in more detail here. The operator chair 1 may also be equipped with wheels so that it can be easily moved to the desired location and parked.

Usually, an operator chair 1 has two armrest devices 2, one on the left side and one on the right side. The armrest devices 2 may be mounted with a clamp 12 on the operator chair 1 using a tool rail 11 on each side. The base position on the rail 11 in the X direction of the complete armrest device 2 can manually be set by sliding the armrest device 2 on the tool rail 11 to the desired position and then locking it with a rotating knob 25.

Both armrest devices 2 may have the same degrees of freedom of automatic movement the X-, Y- and Z directions as described later but can also manually be set individually with variable resistance at the first rotation joint 3 and/ or the second rotation joint 4. This to suit the body constitution and basic work position of an operator. For example, when an operator needs to move in and out of the chair 1 in a sterile state, there is an advantage if the armrests device 2 can be fixed with selectable resistance in both first rotation joint 3 and second rotation joint 4. This means that the operator can move the armrests plate 2a to the sides or towards him or her with the elbows while the rest of the armrest device 2 maintain in its position. By independently being able to choose level of resistance in rotation joints 3 or 4 the complete armrest device 2 has extra movement flexibility in addition to the automatic adjustment features of the armrest device 2.

The stand of the armrest device 2 may comprise two parallelograms assembled so that they create a large selectable range of motion for the complete armrest device 2 from low to high position and back and forth. The design is made so that the armrest plate 2a where the operator rests the arm, wrist or hand is moved parallel and maintains its position in relation to the armrest stand regardless of its position. The design of the armrest device 2 may be such that the rotation centre at the second rotation joint 4 is located close to the centre of gravity of the complete armrest stand to create good balance and stability and to make it easy to manoeuvre.

The armrest device 2 itself is pivotable around the centre of rotation in the second rotation joint 4, when attached to the operator chair 1, where the friction of the rotation can be set according to the operators wishes with a rotating knob 23 that affects a friction piece 17. According to an embodiment, the rotation position i.e. the armrest device Z-direction can also automatically be released or locked by the operator together with the X- and Y direction of the armrest device 2 by the first control mechanism 9.

In the described embodiment, the first control mechanism 9 may be designed as a release lever which may be activated with the thumb, or any other finger. In the activated position it may release the locking of the two gas cylinders 7, 8 as well as the rotation at the second rotation joint 4. The operator can then with low force press down the armrest plate 2a with the wrist and easily set the armrest device 2 to the desired position in large range in X-, Y- and Z direction by moving the armrest plate 2a. The armrest device 2 may be balanced so the operator does not have to pull, hold up or force the armrest device 2 into the selected position. When the operator then releases the first control mechanism 9, the armrest device 2 is locked in the new selected position.

On the armrest plate 2a there may be the second control mechanism 10 and in the described embodiment designed as a release lever which may be activated with the fingers. In the activated position it may release the locking of the third gas cylinder 19 which controls the angle of the armrest plate 2a relative to the rest of the armrest device 2 at an angulation joint 3a. The second control mechanism 10 may act directly on the air inlet of the third gas cylinder 19 via a mechanical link, thereby opening the air inlet of the third gas cylinder 19 when the user press the second control mechanism 10, and lock the air inlet of the third gas cylinder 19 when the user release the second control mechanism 10.

The stand of the armrest device 2 may be based on two parallelograms that comprises a lower arm top 5a and lower arm bottom 5b, a upper arm top 6a and upper arm bottom 6b, an intermediate piece 18 as well as the armrest plate 2a itself which are movable relative to each other around virtual axes of rotation 13, 14, 15 and 16. The lower arm top 5a and the lower arm bottom 5b thus forms a lower arm 5a/ 5b while the upper arm top 6a and the upper arm bottom 6b forms an upper arm 6a/ 6b.

The armrest stand can be described as an upper arm 6a/ 6b, a lower arm 5a/ 5b and an armrest plate 2a where the latter, in analogy with the human arm, functions as the hand and wrist of the armrest device 2. The upper arm 6a/ 6b may be movable up and down, seen from the perspective of the operator, around first axis 13 and second axis 14 which connects with the lower arm 5a/ 5b by intermediate piece 18. The lower arm 5a/ 5b is connected to the upper arm 6a/ 6b via intermediate piece 18 at third axis 15 at the top and forth axis 16 at the bottom. The lower arm 5a/ 5b moves primarily back and forth from the operator's perspective but also raises and lowers depending on its position. Which thus helps to create a large total range of movement.

In addition to the disclosed X-, Y, and Z adjustment of the armrest device 2 with the first control mechanism 9 and/ or the second control mechanism 10 to adjust the angle of the armrest plate 2a, the armrest plate 2a can also be adjusted out and in towards the operator by rotation around the first rotating joint 3 with selectable friction or locked completely. The latter is a manual control like the second rotation joint 4 where the armrest device 2 is attached to the tool rail 11 of the chair 1.

The second rotation joint 4 may comprise a bearing axis around which the armrest device 2 rotates. The said rotation joint 4 may be attached to the clamp 12 which in turn may be used to mount the armrest device 2 on the tool rail 11 of the chair 1. The rotation resistance in the Z direction can be regulated for the desired inertia rotational in the Z direction with a rotating knob 23 and is set in a basic setting by the user and is maintained at that level thereafter. The rotational resistance is created by the friction piece 17 which continuously adjusts the inertia of the movement depending on how the rotating knob 23 is set.

In addition to adjustable resistance in the rotation movement in Z direction, the armrest device 2 can also be locked in selectable rotation positions via locking device consisting of ring gear piece 24 and locking pin 22. The locking pin 22 can run in the ring gear piece 24 when the armrest device 2 is rotated around the second rotation joint 4 and may be activated or deactivated together with the first gas cylinder 7 and the second gas cylinder 8 with the first control mechanism 9 via the lock/ release system 21. The rotation in Z direction may be mechanically blocked in the non-activated state so that the rotation movement is locked. When the first control mechanism 9 is activated, the hydraulic piston or wire of the lock/ release system 21 releases a latch, and the locking pin 22 is allowed to run in the ring gear piece 24.

The positions in the ring gear piece 24 are fixed in fine steps within the intended Z direction range of motion of the armrest device 2. This locking function is activated or deactivated at the same time as the first gas cylinder 7 and/ or the second gas cylinder 8 with the first control mechanism 9. This means that the armrest device 2 is released in all directions X-, Y- and Z direction at the same time when the first control mechanism 9 is activated and is locked in the desired position when the first control mechanism 9 is deactivated. The automatic locking function in Z direction can be permanently disengaged as a basic function and thus allow the rotation to be influenced only via the friction brake operated with rotating knob 23, according to the operator's wishes.

The armrest plate 2a is attached at the first axis of rotation 13 with the first rotating joints 3 and the angulation joint 3a that enables angulation of the armrest plate 2a which make it movably adjustable in two joints in relation to the armrest device 2 comprising the lower arm top/ bottom 5a/ 5b, the upper arm top/ bottom 6a/ 6b and the intermediate piece 18. The up and down movement of the armrest plate 2a may take place over the first rotation joint 3 and be activated with the second control mechanism 10. The armrest plate 2a may be locked at the desired angle by the lockable third gas cylinder 19. The optional rotation sides inwards and outwards towards takes place over first rotation joint 3 that has a similar friction control as second rotation joint 4. The rotation resistance can be regulated to the desired inertia rotational with a rotating knob 20 and can range from loose to fully fixed in some embodiments.

The lockable first and second gas cylinders 7 and 8 are regulated with the first control mechanism 9 and said gas pistons can both be activated simultaneously. The lockable first and second gas cylinders 7 and 8 have a built-in lock that makes them completely fixed in position when not activated by the first control mechanism 9 via the lock/ release system 21 and this keeps the armrest device 2 stable and safe in the selected position. When released, they have a tailored pressure that counteracts the own weight of the armrest stand itself. The first gas cylinder 7 mainly promotes back and forth movement and the second gas cylinder 8 mainly promotes an up and down movement. The stroke length of the gas cylinders 7 and 8 is so adapted that they promote an extensive range of motion from low to high and back and forth, both adjustable. In addition to the first and second gas cylinders 7 and 8, a rotation stop at the second rotation joint 4, for example embodied as a locking pin 22 acting on a ring gear piece 24 may also be activated together with operation of the first control mechanism 9. Thereby the entire armrest device 2 is free floating or locked in the X-, Y- and Z direction at the same time.

To achieve a smooth movement of the armrest device 2 with the first and second gas cylinders 7 and 8 set into unlocked mode, the gas pressure of the respective gas cylinders 7, 8 may be calibrated with each other, depending on for example weight distribution of the particular armrest device 2, weigh of the supported body part, etc.

The first and second gas cylinders 7 and 8 and the rotation control/ rotation stop 22 at the second rotation joint 4 may be jointly regulated with the first control mechanism 9. In the preferred embodiment said first control mechanism 9 is attached to the lock/ release system 21 wherein either hydraulic pistons, or wires, simultaneously activate and deactivate the first and second gas cylinders 7 and 8 and the rotation stop 22.

The first control mechanism 9 in the preferred embodiment is placed under the armrest plate 2a and activated and deactivated by an operator's thumb while the wrist can remain resting on the armrest plate 2a.

The angle of the armrest plate 2a in relation to the armrest device 2 can in addition be adjusted up and down +/- 35 degrees. The angle adjustment may be operated with the fingertips activating the second control mechanism 10 while the operator's wrist can remain resting on the armrest plate 2a. When the second control mechanism 10 is activated, the third gas cylinder 19 having a tailored pressure is released and makes it possible to set the desired angle on the armrest plate 2a. When the second control mechanism 10 is deactivated, the angle of the armrest plate 2a is locked in the selected position. The angle adjustment of the armrest plate 2a may take place independently of the armrest device's other adjustment in X-, Y- and Z direction, by adjustment of the second control mechanism 10. The angle of the armrest plate 2a is maintained when the other positions of the armrest device 2 in X- Y- and Z direction is changed via the first control mechanism 9, because the armrest device 2 then moves in parallel.

## Claims

1. An armrest device (2) for an operator chair (1), which armrest device (2) comprises:
an armrest plate (2a);
an upper arm (6a, 6b);
a lower arm (5a, 5b);
a clamp (12) for attaching the armrest device (2) to a support structure of the operator chair (1);
a plurality of joints (3, 3a, 4, 13, 14, 15, 16) joining the armrest plate (2a), the upper arm (6a, 6b), the lower arm (5a, 5b), and the clamp (12) with each other;
a first gas cylinder (7) configured to enable movements of the inclination of the lower arm (5a, 5b) when set in a released mode, and to disable said movements when set in a locked mode; and
a second gas cylinder (8) configured to enable movements of the inclination of the upper arm (6a, 6b) when set in the released mode, and to disable said movements when set in a locked mode;
a lock/ release system (21) configured to simultaneously set the first gas cylinder (7) and the second gas cylinder (8) in either released mode, or locked mode;
a first control mechanism (9) connected to the lock/ release system (21), wherein the first control mechanism (9) is located at the armrest plate (2a) for user operation without removing a hand of the user from the armrest plate (2a); and wherein the first control mechanism (9) is configured to via the lock/ release system (21) simultaneously set the first gas cylinder (7) and the second gas cylinder (8) in either released mode, or locked mode.

2. The armrest device (2) according to claim 1, wherein the first gas cylinder (7) is configured to allow adjustment of the inclination of the lower arm (5a, 5b) when set in the released mode; and wherein the second gas cylinder (8) is configured to allow adjustment of the inclination of the upper arm (6a, 6b) when set in the released mode.

3. The armrest device (2) according to any one of claims 1-2, wherein the lower arm (5a, 5b) comprises a lower arm top (5a) and lower arm bottom (5b) arranged in a lower parallelogram and the upper arm (6a, 6b) comprises an upper arm top (6a) and an upper arm bottom (6b), arranged in an upper parallelogram.

4. The armrest device (2) according to any one of the preceding claims, further comprising a rotation joint (4) at the clamp (12) of the armrest device (2), wherein the rotation joint (4) is allowed to rotate when set in a released mode, and to disallow rotation when set in a locked mode; and wherein the lock/ release system (21) and the first control mechanism (9) are configured to set the rotation joint (4) in either released mode, or locked mode.

5. The armrest device (2) according to claim 4 wherein the lock/ release system (21) and the first control mechanism (9) are configured to simultaneously set the first gas cylinder (7), the second gas cylinder (8) and the rotation joint (4) in either released mode, or locked mode.

6. The armrest device (2) according to any one of claims 4-5 wherein the rotational resistance of the rotation joint (4) is adjustable.

7. The armrest device (2) according to any one of the preceding claims, further comprising:
a third gas cylinder (19), configured to enable tilt angle adjustment of the armrest plate (2a);
a second control mechanism (10) located at the armrest plate (2a) for adjusting tilt angle of the armrest plate (2a) by setting the third gas cylinder (19) in either released mode, wherein the tilt angle of the armrest plate (2a) is adjustable; or in locked mode, wherein the tilt angle of the armrest plate (2a) is locked.

8. The armrest device (2) of any preceding claim, wherein the clamp (12) is configured to be removably attached to a rail (11) of the support structure.

9. The armrest device (2) of any preceding claim, wherein the lock/ release system (21) comprises a hydraulic system.
